# EUROPEAN PATENT APPLICATION

(11) **EP 1 602 415 A1**
(43) Date of publication of application: **07.12.2005**
(21) Application number: 04013271.4
(22) Date of filing: 04.06.2004
(51) Int. Cl.: B05C 17/005, B65D 81/32, A61C 9/00

(54) **Syringe for a multi-component paste**

(71) Applicant: 3M ESPE AG, 82229 Seefeld (DE)
(72) Inventor: Pauser, Helmut, 86911 Diessen (DE)

(57) **Abstract**

The present invention relates to a syringe for storage, mixing and dispensing of multi-component paste materials, and more precisely to a syringe for two or more components of a paste material which are to be mixed together.

## Description

### BACKGROUND OF THE INVENTION

### TECHNICAL FIELD

The present invention relates to a syringe for storage, mixing and dispensing of multi-component paste materials, and more precisely to a syringe for two or more components of a paste material which are to be mixed together.

### DESCRIPTION OF THE RELATED ART

Known syringes require an activation step, disassembly/assembly of parts and usually they do not provide a complete extrusion of the paste.

### SUMMARY OF THE INVENTION

The present invention provides the advantage that no activation or other preparation step is necessary prior to use and at the same time the syringe allows complete extrusion of the paste by collapsing the mixer. Activation, mixing and paste extrusion as well as collapsing the mixer can be done in an all in one movement. Furthermore the length of the syringe is within usual dimensions of syringes which have proven for optimum handling.

In a first aspect, the present invention provides a three-step function of the plunger or piston assembly whereas switching from the first to the third step occurs automatically and during an "all in one" movement.

In the first step the syringe is activated which means that the chambers containing the paste components are opened.

In the second step the paste components are extruded through a mix tip containing a static mixer and through a dispensing nozzle.

And finally in the third step the mixer is compressed to extrude all the remaining paste out of the mix tip.

In a second aspect, the present invention relates to a concentric arrangement of the paste chambers and a closure plug which interact in a manner that only one plug is used to seal all chambers to encapsulate the stored pastes and to open all chambers at the same time when it is displaced. A further advantage of the concentric arrangement of the chambers is the potential to easily make a syringe for multi-component pastes as well as for two-component and multi-component pastes without changing the syringe design.

In a further aspect, the present invention relates to a syringe for two or more components of a material which are to be mixed together, comprising:
- a first component chamber for containing a first component and a second component chamber for containing a second component, each component chamber comprising a chamber outlet;
- a first piston for movement within the first component chamber and a second piston for movement within the second component chamber;
- a mixing tip comprising a front end with a dispensing opening and a rear end with an inlet opening, the rear end being connected to the chamber outlets;
- a static mixer arranged in the mixing tip;
- a plug comprising a first flow channel for the first component and a second flow channel for the second component, the plug being movably arranged in the first component chamber in such a manner that when the plug is in a first position, the chamber outlets are closed and when the plug is in a second position, the inlet opening of the mixing tip is connected via the first and the second flow channels to the first and the second chamber outlets respectively.

Further preferred features and embodiments of the invention are described in the claims.

It may be provided that the first component chamber is concentrically arranged inside the second component chamber.

### DETAILED DESCRIPTION OF PREFERRED EMBODIMENTS

Preferred embodiments of the invention are described in more detail below with reference to the attached drawings, which are by way of example only.

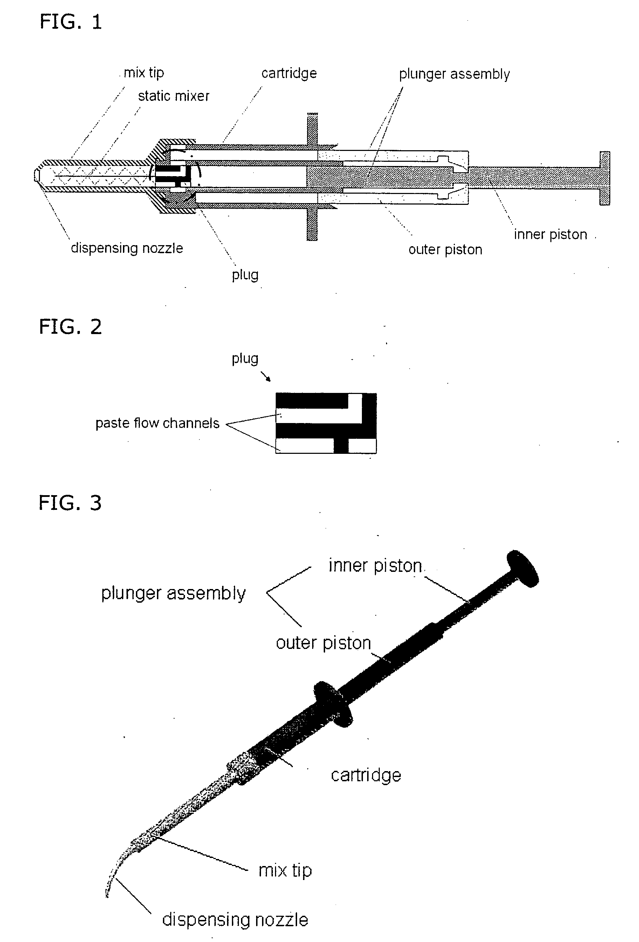

In a preferred embodiment the syringe comprises a cartridge, a plug, a piston assembly, and a mix tip with a static mixer (FIG. 1-4).

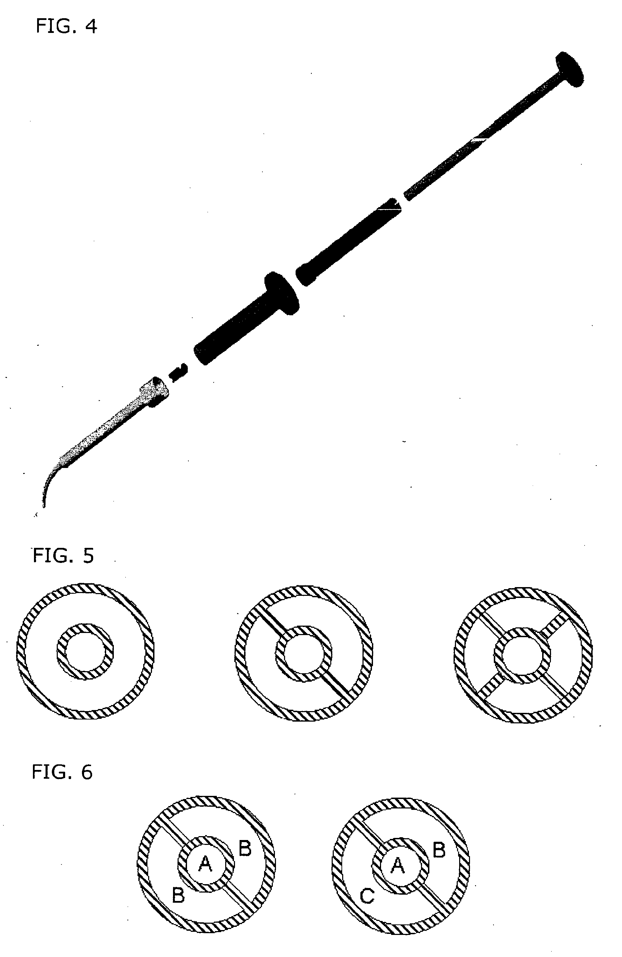

The cartridge is generally formed by two concentric arranged barrels (FIG. 5) forming the component chambers for storing the paste components. The inner barrel acts as component chamber for a first paste component, the annular gap between the inner and outer barrel acts as a component chamber for a second paste component. The annular gap may be easily divided into two or more component chambers so as to create a syringe for the respective number of components.

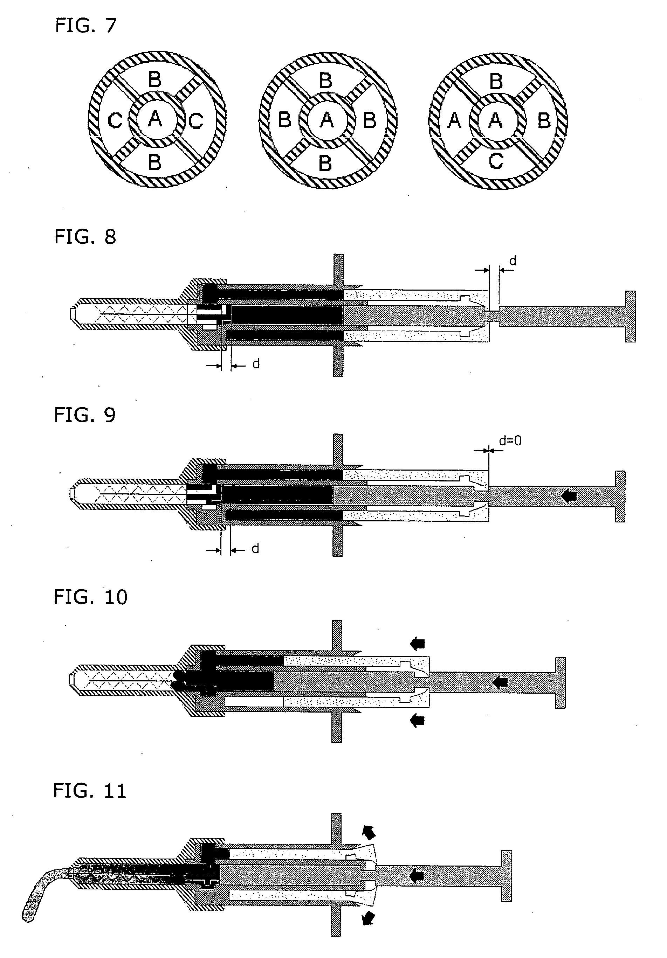

The concentric arrangement of the barrels allows a variety of different configurations (FIG. 5) for the paste materials filled into the syringe. The same cartridge design may be used for materials of different numbers of components: For example, the three-barrel design shown in the middle picture of FIG. 5 may be used for a two-component material (left picture of FIG. 6) and for a three-component material (right picture of FIG. 6). Or, as another example, the five-barrel design shown in the right picture of FIG. 5 may be used for a two-component material (middle picture of FIG. 7) and for a three-component material (left and right pictures of FIG. 7) as well as for a four-component and a five-component material (not shown).

A further advantage of the described concentric multi-component cartridge results from using a two-component paste material with a multi-barrel cartridge, for example a four-barrel cartridge. In this case the components can be arranged in an alternating order so as to achieve premixing via the cartridge design. In this way one mixing element of the static mixer can be saved.

### GENERAL FUNCTION (FIG. 8-12)

The inner piston can be pushed forward over a distance d (FIG. 8) until it links automatically with the outer piston (FIG. 9). This short movement over the distance d causes the plug to move forward by the same distance d thus opening the chambers containing the paste.

Because of the now existing linkage between the inner and outer piston the whole piston assembly (inner and outer piston) moves forward upon displacement of the inner piston (FIG. 10). This way the paste components are extruded out of the inner and outer chambers simultaneously. The paste components flow through the flow channels of the plug into the mix tip, where they are mixed, and finally through the dispensing nozzle until the outer piston has reached its end position.

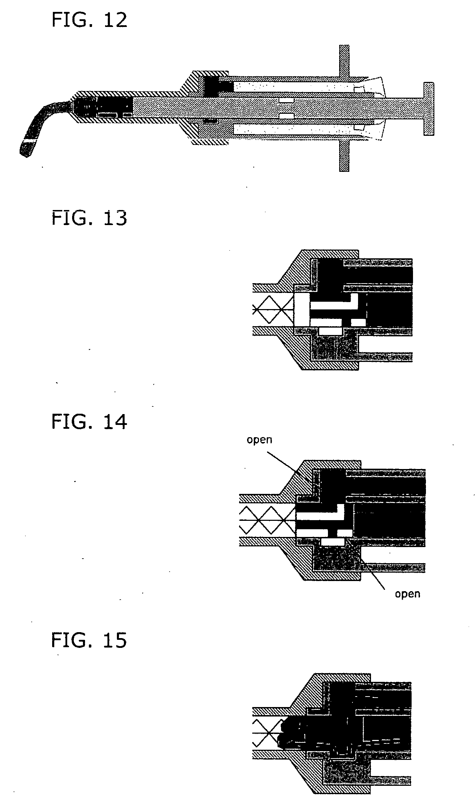

Shortly before or at the same time the outer piston has reached the end position the linkage between inner and outer piston is released (FIG. 11) and the inner piston moves separately and extrudes the paste in the mix tip while the static mixer is compressed (FIG. 12).

### ONE PLUG USED AS VALVE (FIG. 13-15)

Only one plug may be used as a valve to seal and open the outlets of all paste chambers. If the plug, starting from the initial position in which all chambers outlets are closed (FIG. 13), is displaced by the distance d (e.g. as mentioned above) the syringe will be activated so that all chamber outlets are opened (FIG. 14). Then the paste can be extruded through channels (FIG. 15) which are arranged within the plug.

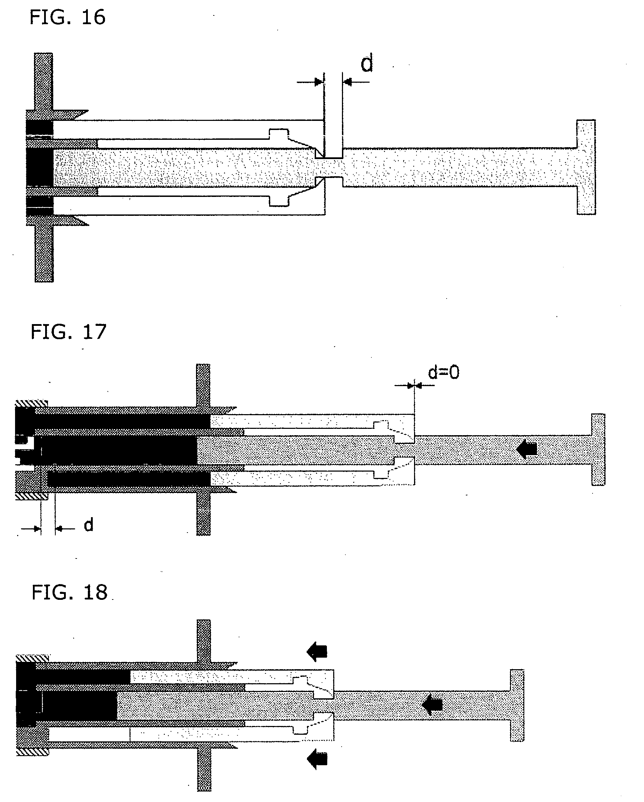

### LINKAGE BETWEEN INNER AND OUTER PISTON (FIG. 16-20)

To achieve activation, extrusion and mixer compression is actuated within an all in one movement, a special linkage between the inner and outer pistons is proposed.

The initial position of both pistons and the position after the syringe has been activated is shown in FIG. 16+17, respectively.

The outer piston forms a spring sleeve which engages with a recess arranged at the inner piston. The recess allows the inner piston to be displaced independently from the outer piston by a distance d (FIG. 16). When the inner piston is displaced the paste in the inner chamber as well as the plug are moved forward (FIG. 17). During this step the plug opens all chamber outlets.

After the inner piston has been displaced by the distance d further movement will cause the outer piston to move together with the inner piston (FIG. 18) because of the engagement of the spring sleeve with the recess in the inner piston. In this way the paste can be extruded from the inner and outer chambers simultaneously.

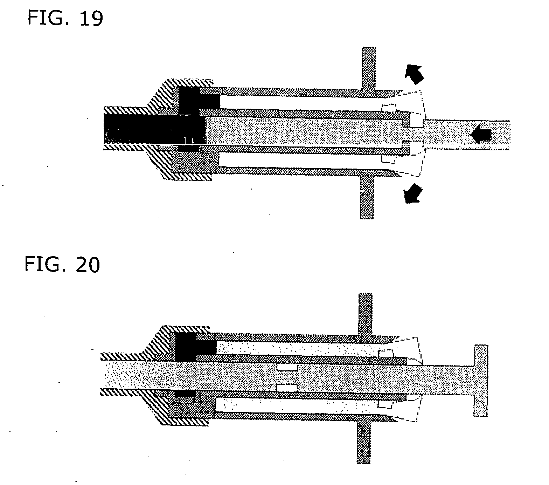

As soon as the outer piston of the piston assembly has nearly reached its final position, the spring sleeve is released from the recess in the inner piston (FIG. 19) and the inner piston can be further moved separately.

Releasing the spring sleeve is actuated by the inner barrel which penetrates the piston assembly thus displacing the springs when the piston assembly approaches its final position.

After the spring sleeve is disengaged from the inner piston separate displacement of the inner piston will cause the mixer to be compressed and all the paste to be extruded from the inner barrel (FIG. 20).

The present invention has now been described with reference to several embodiments thereof. It will be apparent to those skilled in the art that many changes can be made in the embodiments described without departing from the scope of the present invention. Thus the scope of the present invention should not be limited to the structures described in this application, but only by structures described by the language of the claims and the equivalents of those structures.

## Claims

1. Syringe for two or more components of a material which are to be mixed together, comprising:
- a first component chamber for containing a first component and a second component chamber for containing a second component, each component chamber comprising a chamber outlet;
- a first piston for movement within the first component chamber and a second piston for movement within the second component chamber;
- a mixing tip comprising a front end with a dispensing opening and a rear end with an inlet opening, the rear end being connected to the chamber outlets;
- a static mixer arranged in the mixing tip;
- a plug comprising a first flow channel for the first component and a second flow channel for the second component, the plug being movably arranged in the first component chamber in such a manner that when the plug is in a first position, the chamber outlets are closed and when the plug is in a second position, the inlet opening of the mixing tip is connected via the first and the second flow channels to the first and the second chamber outlets respectively.

2. Syringe according to claim 1, wherein the first component chamber is concentrically arranged inside the second component chamber.
